# EUROPÄISCHE PATENTSCHRIFT

(11) **EP 1 865 748 B1**
(45) Veröffentlichungstag und Bekanntmachung des Hinweises auf die Patenterteilung: **19.08.2009**
(21) Anmeldenummer: 07108595.5
(22) Anmeldetag: 22.05.2007
(51) Int. Cl.: H04R 25/00

(54) **Verfahren zum Erstellen eines individuellen Hörgeräteprogramms**
Method for creating an individual hearing aid programme
Procédé destiné à la fabrication d'un programme d'appareil auditif individuel

(30) Priorität: 07.06.2006 DE 102006026489
(43) Veröffentlichungstag der Anmeldung: 12.12.2007
(73) Patentinhaber: Siemens Audiologische Technik GmbH, 91058 Erlangen (DE)
(72) Erfinder: Giese, Ulrich, 91052 Erlangen (DE); Grafenberg, Esfandiar, 91090 Effeltrich (DE)
(74) Vertreter: Maier, Daniel Oliver

(56) Entgegenhaltungen:
- WO-A-20/05125280
- DE-A1- 4 227 826
- US-A1- 2005 086 058

## Beschreibung

Die vorliegende Erfindung betrifft ein Verfahren zum Erstellen eines individuellen Hörgeräteprogramms zur Verbesserung von Klang und Sprachverständlichkeit.

Studien belegen, dass für Hörgeräteträger die Sprachverständlichkeit und der Klang beim Fernsehen häufig nicht zufriedenstellend sind. Der Grund hierfür liegt darin, dass die Hörgeräteprogramme in der Regel nur pauschal für die Hörsituation "Fernsehen" entwickelt sind und dabei nicht auf individuelle Hörsituationen eingegangen werden kann. Zum anderen sind die Hörverluste der Hörgeräteträger derart unterschiedlich, dass auch diesbezüglich keine Individualisierung im Hinblick auf die Sprachverständlichkeit von Fernsehsendungen stattfinden kann. Die Hörsituation "Fernsehen" wird aber von den Hörgeräteträgern als sehr wichtig angesehen.

Derzeit bietet praktisch jeder Hörgerätehersteller ein Hörgerätesignalverarbeitungsprogramm für das Betrachten von Fernsehsendungen an. Diese Hörgeräteprogramme sind in ihrem Frequenzgang pauschal etwas gegenüber anderen Hörgeräteprogrammen geändert. Hieraus ergibt sich aber in vielen Fällen ein unzureichender Klang und eine unzureichende Sprachverständlichkeit.

Aus der Druckschrift EP 1 320 282 A2 ist ein Verfahren zum Aufzeichnen von Informationen in einem Hörgerät bekannt. Dieses Verfahren bringt dem Entwickler von Hörgeräten den Vorteil, dass die Wahl der automatischen Betriebseinstellungen in Abhängigkeit von den wirklichen akustischen Umgebungen getestet und kontrolliert werden kann.

Darüber hinaus beschreibt die Druckschrift DE 199 28 115 A1 ein Verfahren zum Anpassen von Hörgeräteparametern. Dabei werden mittels eines Audio-Aufzeichnungsgeräts unterschiedliche Audio-Informationen in unterschiedlichen Situationen erfasst und zu einem späteren Zeitpunkt zum Anpassen des Hörgeräts verwendet.

Die Druckschrift WO2005/125280 A2 gibt ein Verfahren und eine Vorrichtung zur Simulation eines Hörgeräts bzw. eines Hörgerätebetriebs an. Dieses Verfahren bzw. diese Vorrichtung wird vor dem Kauf eines Hörgeräts zur Bestimmung des Hörverlustes eines Hörgeschädigten verwendet.

Die Aufgabe der vorliegenden Erfindung besteht somit darin, ein Verfahren anzugeben, mit dem es möglich ist, Hörgeräte so einzustellen, dass die Sprachverständlichkeit und der Klang bei Medienwiedergaben verbessert ist.

Erfindungsgemäß wird diese Aufgabe gelöst durch ein Verfahren zum Erstellen eines individuellen Hörgeräteprogramms durch Aufnehmen von Akustiksignalen an einem Aufnahmeort als Basisaufnahme auf einen Datenträger, gleichzeitiges Aufnehmen der Akustiksignale mit einem ersten Mikrofon als erste Aufnahme an dem Aufnahmeort unabhängig von der Basisaufnahme, Abspielen des Datenträgers auf einem individuellen Wiedergabegerät und/oder in einer individuell vorgegebenen Umgebung, die von dem Aufnahmeort verschieden ist, erneutes Aufnehmen des von dem Datenträger abgespielten Signals mit einem zweiten Mikrofon als zweite Aufnahme, Bilden einer Verknüpfung der ersten Aufnahme und der zweiten Aufnahme, Einstellen eines Hörgeräteprogramms in Abhängigkeit von der Verknüpfung.

Erfindungsgemäß kann somit der Klang und die Sprachverständlichkeit bei der medialen Wiedergabe individuell verbessert werden, da individuelle Gegebenheiten bei der Einstellung des Hörgeräts berücksichtigt werden.

Vorzugsweise handelt es sich bei der Basisaufnahme um eine Film- oder Videoaufnahme. Die Aufnahme kann dann live in einem Studio erfolgen, wobei auch gleichzeitig die Akustiksignale mit dem ersten Mikrofon live in dem Studio aufgenommen werden.

Der Datenträger kann eine DVD sein, die mit einem individuellen Fernsehgerät als individuelles Wiedergabegerät abgespielt wird. Selbstverständlich können auch andere Speichermedien wie CDs, Festspeicher, Bänder usw. als Datenträger zur Wiedergabe an einem individuellen Fernsehgerät eingesetzt werden.

Besonders vorteilhaft ist es, wenn das erste Mikrofon und das zweite Mikrofon dasselbe oder das gleiche Hörgerätemikrofon ist/sind. Damit kann für einen Hörgeräteträger der genaue Unterschied zwischen der akustischen Situation in einem Studio und der individuellen akustischen Situation beispielsweise in einem Wohnzimmer ermittelt und berücksichtigt werden.

Entsprechend einer Weiterbildung des erfindungsgemäßen Verfahrens ist die erste und die zweite Aufnahme jeweils eine Langzeitspektralaufnahme. Damit lassen sich die akustischen Bedingungen bei Aufnahme und Wiedergabe besser allgemein beurteilen.

Darüber hinaus kann ein Enthallungs-Algorithmus des Hörgeräteprogramms in Abhängigkeit von der Verknüpfung individuell parametrisiert werden. Auf diese Weise kann bei der Erstellung eines individuellen Hörgeräteprogramms speziell auf die Halleigenschaften der Umgebung eines Hörgeräteträgers eingegangen werden.

Darüber hinaus kann in vorteilhafter Weise vorgesehen sein, dass die Verknüpfungsdaten über eine Schnittstelle direkt in ein Hörgerät eingegeben werden. Somit lassen sich beispielsweise Filter in einem Hörgerät sehr rasch konfigurieren.

Bei der Verknüpfung zwischen der ersten und zweiten Aufnahme kann es sich um einen Quotienten oder eine Differenz der beiden Aufnahmen handeln. Es können aber auch andere vernünftige mathematische Verknüpfungen verwendet werden, um den Unterschied zwischen einer Standardhörsituation und einer individuellen Hörsituation für die Hörgerätewiedergabe zu berücksichtigen.

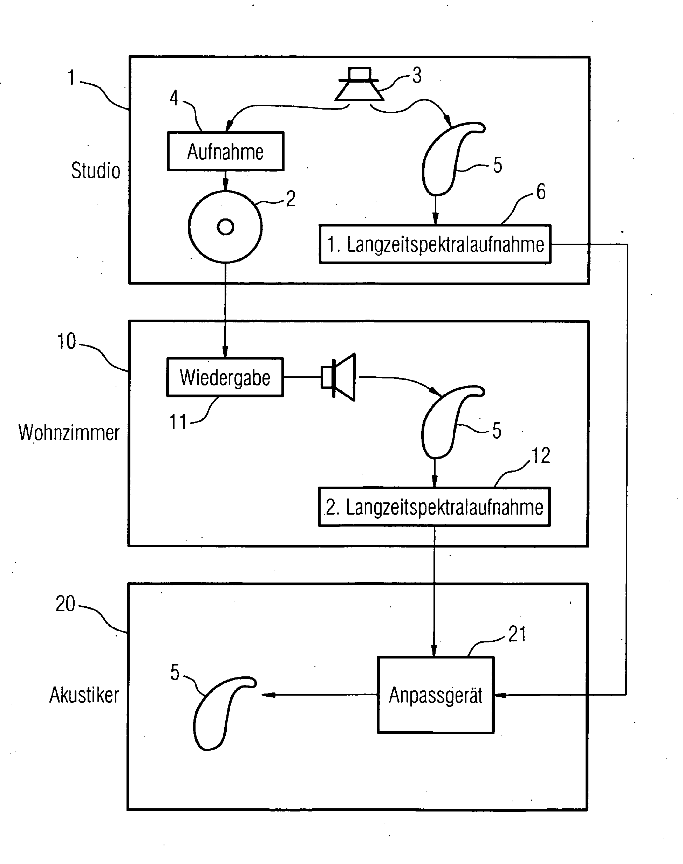

Die vorliegende Erfindung wird nun anhand der beigefügten Zeichnung näher erläutert, die eine Prinzipskizze eines Ausführungsbeispiels eines erfindungsgemäßen Verfahrens wiedergibt.

Die nachfolgend näher geschilderten Ausführungsbeispiele stellen bevorzugte Ausführungsformen der vorliegenden Erfindung dar.

Das Hörgeräteprogramm für Fernsehen soll für einen Hörgeräteträger individuell eingestellt werden. Hierzu wird zunächst in einer akustischen Standardsituation in einem Studio 1 eine Aufnahme eines Fernsehprogramms auf einen Datenträger, insbesondere eine DVD 2 gemacht. Die Signalquelle ist hier durch einen Lautsprecher 3 symbolisiert, der den Schall für ein Aufnahmegerät 4 generiert. Das Aufnahmegerät 4 liefert dann die DVD 2.

Gleichzeitig werden die akustischen Ausgangssignale des Lautsprechers 3 im Studio 1 über ein Hörgerät 5 mitgeschnitten. Dabei befindet sich das Hörgerät 5 an einem Kunstkopf, und zwar in der Nähe der Schläfe, wo es üblicherweise auch vom Hörgeräteträger getragen wird. Damit entsprechen die akustischen Umgebungsbedingungen in etwa denen, wie sie ein Hörgeräteträger tatsächlich vorfinden würde.

Der über das Hörgerät 5 aufgenommene Mitschnitt wird statistisch gemittelt. Daraus resultiert eine erste Langzeitspektralaufnahme 6. Diese spiegelt die akustische Umgebung in dem Studio 1 während der Aufnahme wieder.

Die DVD 2 wird nun im Wohnzimmer 10 eines Hörgeräteträgers abgespielt. Das Wohnzimmer 10 steht hier für eine individuelle akustische Umgebung, die prinzipiell auch jeder andere Raum oder ein Kopfhörer sein kann. Wesentlich ist, dass die Wiedergabe über ein individuelles Gerät des Hörgeräteträgers in seiner vertrauten Umgebung mit der gewohnten Lautstärke erfolgt. Im konkreten Fall handelt es sich bei dem Wiedergabegerät 11 um den individuellen Fernseher. Dieser gibt entsprechend der Aufnahme auf der DVD 2 ein entsprechendes akustisches Ausgangssignal in dem Wohnzimmer 10 wieder.

Der Hörgeräteträger wird Fernsehsendungen typischerweise immer an einem bestimmten Ort in seinem Wohnzimmer 10 verfolgen. Sein Ohr befindet sich dann an einer entsprechend vorgegebenen Stelle. An dieser Stelle wird bei der Wiedergabe der DVD 2 der Schall über das gleiche Mikrofon, hier das Hörgerätemikrofon erneut aufgenommen. Für diese Zwecke kann eine Hörgeräteleihgabe von einem Akustiker, das eingebaute Mikrofon in einer Fernbedienung oder direkt ein Hörgerätemikrofon verwendet werden.

Die mit Hilfe dieses Mikrofons beziehungsweise des Hörgeräts 5 erneut durchgeführte Aufnahme spiegelt die Einflüsse des individuellen Fernsehers und die akustischen Eigenschaften des Fernseh- beziehungsweise Wohnzimmers des Hörgeräteträgers wieder. Diese erneute Aufnahme erfolgt beispielsweise digital in dem Format MP3. Neben der Wiedergabe des Films beziehungsweise Videos der DVD 2 können in dem Wohnzimmer 10 beziehungsweise der individuellen Umgebung auch andere definierte Testsignale abgespielt und über das Hörgerät 5 beziehungsweise ein entsprechendes anderes Mikrofon wieder aufgenommen werden. Aus diesen im Wohnzimmer 10 durchgeführten Aufnahmen wird ähnlich wie aus den Aufnahmen in dem Studio 1 eine zweite Langzeitspektralaufnahme 12 erstellt.

Die beiden Langzeitspektralaufnahmen 6 und 12 werden nun einem Akustiker 20 übermittelt. Diese Übermittlung kann beispielsweise auch über ein Datennetz, gegebenenfalls online erfolgen. Der Akustiker subtrahiert in einem Anpassgerät 21 die beiden Langzeitspektralaufnahmen voneinander, die einmal live und einmal vom Fernseher des Hörgeräteträgers stammen, um auch Daten über die akustische Situation des Hörgeräteträgers in seiner gewohnten Umgebung zu gewinnen. Aus diesen Daten wird der Frequenzgang des Hörgeräts 5 in dem für Fernsehen optimierten Hörgeräteprogramm individuell angepasst. Darüber hinaus können aus der Aufnahme spezieller Testsignale individuelle Einstellparameter für Enthallungs-Algorithmen extrahiert werden.

Für die Anpassung gibt der Akustiker die Daten ähnlich wie bei einer RECD (Real Ear to Coupler Difference), mit der man bei der heutigen Software den Frequenzgang dem Volumen des Gehörgangs (z. B. bei Kindern) anpassen kann, in die Software ein. Die Daten können aber auch über beispielsweise einen USB-Anschluss automatisch eingespeist werden. Ist dann das Hörgerät auf diese Weise individuell angepasst, so verbessert sich der Klang und die Sprachverständlichkeit beim Fernsehen für den Hörgeräteträger.

## Patentansprüche

1. Verfahren zum Erstellen eines individuellen Hörgeräteprogramms durch
- Aufnehmen von Akustiksignalen an einem Aufnahmeort (1) als Basisaufnahme auf einen Datenträger (2),
- gleichzeitiges Aufnehmen der Akustiksignale mit einem ersten Mikrofon als erste Aufnahme an dem Aufnahmeort (1) unabhängig von der Basisaufnahme,
- Abspielen des Datenträgers (2) auf einem individuellen Wiedergabegerät (11) und/oder in einer individuell vorgegebenen Umgebung (10) , die von dem Aufnahmeort (1) verschieden ist,
- erneutes Aufnehmen des von dem Datenträger (2) abgespielten Signals mit einem zweiten Mikrofon als zweite Aufnahme,
- Bilden einer Verknüpfung der ersten Aufnahme und der zweiten Aufnahme,
- Einstellen eines Hörgeräteprogramms in Abhängigkeit von der Verknüpfung.

2. Verfahren nach Anspruch 1, wobei die Basisaufnahme eine Film- oder Videoaufnahme ist.

3. Verfahren nach Anspruch 1 oder 2, wobei der Datenträger (2) eine DVD ist, die mit einem individuellen Fernsehgerät als individuelles Wiedergabegerät (11) wiedergegeben wird.

4. Verfahren nach einem der vorhergehenden Ansprüche, wobei das erste Mikrofon und das zweite Mikrofon dasselbe oder das gleiche Hörgerätemikrofon ist.

5. Verfahren nach einem der vorhergehenden Ansprüche, wobei die erste und die zweite Aufnahme jeweils eine Langzeitspektralaufnahme (6, 12) ist.

6. Verfahren nach einem der vorhergehenden Ansprüche, wobei ein Enthallungs-Algorithmus des Hörgeräteprogramms in Abhängigkeit von der Verknüpfung individuell parametrisiert wird.

7. Verfahren nach einem der vorhergehenden Ansprüche, wobei die Verknüpfungsdaten über eine Schnittstelle direkt in ein Hörgerät (5) eingegeben werden.

8. Verfahren nach einem der vorhergehenden Ansprüche, wobei die Verknüpfung ein Quotient oder eine Differenz ist.

## Claims

1. Method for generating an individual hearing device program by
- recording acoustic signals at a recording site (1) as a basic recording on a data medium (2),
- simultaneously recording the acoustic signals as a first recording with a first microphone at the recording site (1) independently of the basic recording,
- playing back the data medium (2) on an individual playback device (11) and/or in a predetermined individual environment (10) that is different to the recording site (1)
- re-recording as a second recording with a second microphone the signal played back by the data medium (2)
- forming a connection between the first recording and the second recording,
- adjusting a hearing device program as a function of the connection.

2. Method according to claim 1, with the basic recording being a film or video recording.

3. Method according to claim 1 or 2, with the data medium (2) being a DVD which is played back with an individual television receiver as an individual playback device (11).

4. Method according to one of the preceding claims, with the first microphone and the second microphone being one and the same or being an identical hearing device microphone.

5. Method according to one of the preceding claims, with the first and the second recording each being a long-term spectral recording (6, 12).

6. Method according to one of the preceding claims, with a dereverberation algorithm of the hearing device program being individually parameterised as a function of the connection.

7. Method according to one of the preceding claims, with the connection data being input directly into a hearing device (5) via an interface.

8. Method according to one of the preceding claims, with the connection being a quotient or a difference.

## Revendications

1. Procédé destiné à établir un programme d'appareil auditif individuel par
- enregistrement de signaux acoustiques à un lieu d'enregistrement (1) en tant qu'enregistrement de base sur un support de données (2),
- enregistrement simultané des signaux acoustiques à l'aide d'un premier microphone en tant que premier enregistrement au lieu d'enregistrement (1), indépendamment de l'enregistrement de base,
- reproduction du support de données (2) sur un appareil de reproduction individuel (11) et/ou dans un environnement (10) défini individuellement, qui est différent du lieu d'enregistrement (1),
- nouvel enregistrement du signal reproduit par le support de données (2) à l'aide d'un second microphone, en tant que second enregistrement,
- formation d'une liaison du premier enregistrement et du second enregistrement,
- réglage d'un programme d'appareil auditif en fonction de la liaison.

2. Procédé selon la revendication 1, l'enregistrement de base étant un enregistrement de film ou de vidéo.

3. Procédé selon la revendication 1 ou 2, le support de données (2) étant un DVD qui est reproduit en tant qu'appareil de reproduction individuel (11) à l'aide d'un téléviseur individuel.

4. Procédé selon l'une quelconque des revendications précédentes, le premier microphone et le second microphone étant le même microphone d'appareil auditif ou un microphone d'appareil auditif identique.

5. Procédé selon l'une quelconque des revendications précédentes, le premier et le second enregistrements étant respectivement un enregistrement spectral de longue durée (6, 12).

6. Procédé selon l'une quelconque des revendications précédentes, un algorithme de déréverbération du programme d'appareil acoustique étant paramétré individuellement en fonction de la liaison.

7. Procédé selon l'une quelconque des revendications précédentes, les données de liaison étant entrées directement dans un appareil acoustique (5) par l'intermédiaire d'une interface.

8. Procédé selon l'une quelconque des revendications précédentes, la liaison étant un quotient ou une différence.
